# EUROPEAN PATENT SPECIFICATION

(11) **EP 3 556 766 B1**
(45) Date of publication and mention of the grant of the patent: **31.07.2024**
(21) Application number: 17879807.0
(22) Date of filing: 15.12.2017
(51) Int. Cl.: C07K 7/08, C07K 14/005, C07K 19/00

(54) **USE OF A CELL MEMBRANE PENETRATING PEPTIDE AND INTRACELLULAR DELIVERY CARRIER INCLUDING THE SAME**
ZYTOPLASMATISCHES TRANSDUKTIONSPEPTID UND INTRAZELLULÄRER MESSENGER DAMIT
PEPTIDE DE TRANSDUCTION CYTOPLASMIQUE ET MESSAGER INTRACELLULAIRE LE COMPRENANT

(30) Priority: 16.12.2016 KR 20160172548
(43) Date of publication of application: 23.10.2019
(73) Proprietor: Avixgen Inc., Seoul 06591 (KR)
(72) Inventor: BAEK, Yi Yong, Goyang-si Gyeonggi-do 10375 (KR); KIM, Min Jung, Seoul 08785 (KR)
(74) Representative: Weickmann & Weickmann PartmbB
(86) International application number: PCT/KR2017/014897
(87) International publication number: WO 2018/111051

(56) References cited:
- WO-A1-2005/051419
- WO-A1-2012/118092
- WO-A1-93/20220
- WO-A2-03/060098
- JP-A- H01 224 398
- US-A1- 2008 039 528
- US-A1- 2015 266 939
- DATABASE NCBI [O] 10 February 2013 (2013-02-10), XP055499082, Database accession no. NP _ 687035.1
- DATABASE GenBank [O] 19 February 2016 (2016-02-19), "Putative Gag-pro-pol Poly protein [Murine leukemia virus", XP055499067, Database accession no. AMK06423.1
- PDB: 2L44 A: CHAIN A , C-TERMINAL ZINC KNUCKLE OF THE HIVNCP7, 10 October 2012 (2012-10-10), XP055499063
- PDB: 2IHX_A: CHAIN A , SOLUTION STRUCTURE OF THE ROUS SARCOMA VIRUS NUCLEOCAPSID PROTEIN:UPSI RNA PACKAGING SIGNAL COMPLEX, 9 October 2012 (2012-10-09), XP055499061
- GAJ, THOMAS ET AL.: "Protein Delivery Using Cys2-His2 Zinc-finger Domains", ACS CHEMICAL BIOLOGY, vol. 9, 2014, pages 1662 - 1667, XP055499064

## Description

### [Technical Field]

The present invention relates to the use of an intracellular delivery carrier including a cell membrane penetrating peptide for delivering a cargo into a cell in vitro.

### [Background Art]

The nucleocapsid protein (hereinafter referred to as 'NC') of the human immunodeficiency virus (HIV) plays a functional role in the viral life cycle as well as the structural role for viral growth. The functions are as follows. First, NC peptides are involved in the genomic encapsulation of viruses. This function results from two zinc finger domains consisting of unique CCHC motifs. The domains are known to be highly conserved in all retroviruses and are essential for HIV RNA packaging and infectious virus production. Second, NC peptides are known to promote tRNA primer annealing and strand transfer during viral reverse transcription (RT), suggesting that NC peptides play an important role in viral replication. Third, NC peptides have the nucleic acid chaperone activity necessary for the viral life cycle. Recently, it has been reported that NC peptides play a predetermined role even when the viral DNA is inserted into the host cell chromosome.

Meanwhile, cell penetrating peptides (CPP) are cell membrane penetrating peptides composed of about 10-30 short peptides. Most of them are derived from protein-transduction domain or membrane-translocating sequence. Unlike the general intracellular entry pathway of foreign substances, CPP is known to be capable of transferring DNA and proteins that are known to be unable to pass through cell membranes into cells without damaging the cell membrane. The best-known peptides for CPP are the Tat peptides, which are derived from Human Immuno-deficiency Virus (HIV) and are currently being used in a variety of applications, such as cell therapy and diagnostic reagents. In addition, Penetratin, derived from the DNA-binding domain of a homeodomain transcription factor, is also being used to deliver proteins useful to the human body to the skin. The transportan is a peptide made by fusing some of the peptides isolated from the venom of the wasp called mastoparan with a neuropeptide called galanin and is used as a cell death-inducing peptide by binding with a peptide inducing cell death.

US patent application No. 2008/0039528 relates to techniques for chelation or ejection of Zn²⁺ from zinc finger peptides by contacting the zinc finger peptides with bishydroxamic acid.

International patent application WO 03/060098 relates to a method for determining whether a compound inhibits the binding of a human immunodeficiency virus nucleocapsid 7 polypeptide to HIV-1 RNA. Specific nucleocapsid 7 polypeptide sequences which are useful in this context and variants thereof are shown, such as NCp7 a small basic protein containing two copies of a conserved zinc finger-like domain with the sequence Cys-X2-Cys-X4-His-X4-Cys, designated as the CCHC motif that binds to RNA.

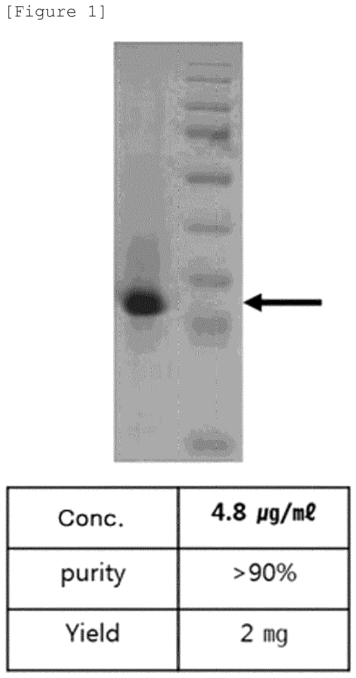

International patent application WO 93/20220 relates to constructs comprising mutant HIV genomes having an alteration in a nucleotide sequence, which is critical for genome RNA packaging. The nucleotide sequence might be the Gag-gene of HIV. Figure 1b shows sequences comprising the parent 5' and 3' CysHis boxes of HIV-1 Gag (pHXB2) and various mutant sequences comprising point mutations.

International patent application WO 2005/051419 relates to retrovirus-like particles having an altered nucleocapsid domain that inhibits packaging of genomic RNA into virus-like particles and a protease having reduced enzymatic activity. Table 1 provides the wild-type sequences from different viruses, which may be altered.

Japanese patent application JP H01 224 398 relates to the synthetic production of antiviral peptides.

International patent application WO 2012/118092 discloses the constructed plasmid pBApo-NC-MazF. The plasmid is a plasmid capable of expressing a fusion protein of NC and MazFD2.

The present inventors have confirmed that NC peptides have cell membrane penetration activity while studying the physiological activity of HIV NC peptides so that NC peptides can be used as a drug delivery carrier capable of delivering intracellular substances, thereby completing the present invention.

### [Detailed Description of the Invention]

### [Technical Problem]

An object of the invention is to provide delivery of a cargo of an object into a cell in vitro using an intracellular delivery carrier.

Still another object of the present invention is to provide an in vitro method for delivering a cargo of an object to be delivered into a cell.

### [Technical Solution]

The invention is defined by the appended claims. In addition, various aspects are described in this document which are intended to illustrate the background of the invention and are related to the invention but not within the scope of the claims.

The present document describes a cell penetrating peptide including an amino acid sequence represented by the following Formula I:

[Formula I] Cys-Xaa₁-Xaa₂-Cys-Xaa₃-Xaa₄-Xaa₅-Gly-His-Xaa₆-Xaa₇Xaa₈-Xaa₉-Cys
in which Xaa₁ is an amino acid selected from the group consisting of Ala, Val, Ile, Leu, Met, Phe, Tyr, Trp, Ser, Thr, Asn and Gln,
in which Xaa₂ is an amino acid selected from the group consisting of Ala, Val, Ile, Leu, Met, Phe, Tyr, Trp, Arg, His, Lys, Asn, Ser, Thr and Gln,
in which Xaa₃ and Xaa₄ are individually an amino acid selected from the group consisting of Asp, Glu, Arg, His, Lys, Ser, Thr, Asn, Gln and Gly,
in which Xaa₅ is an amino acid selected from the group consisting of Asp, Glu, Arg, His, Lys, Ala, Val, Ile, Leu, Met, Phe, Tyr, Trp and Pro,
in which Xaa₆ and Xaa₇ are individually an amino acid selected from the group consisting of Ser, Thr, Asn, Gln, Ala, Val, Ile, Leu, Met, Phe, Tyr and Trp,
in which Xaa₈ is an amino acid of Lys, Ala or Arg, and
in which Xaa₉ is an amino acid selected from the group consisting of Asp, Glu, Ser, Thr, Asn and Gln.

Another aspect described in the present document is an intracellular delivery carrier in which a cargo of an object to be delivered into a cell is conjugated with the end of the cell penetrating peptide.

As used herein, the term "intracellular delivery carrier" refers to a carrier capable of penetrating through the cell membrane and penetrating into the tissue.

As used herein, the term "peptide" or "polypeptide" refers to a linear molecule formed by linking amino acid residues together through peptide bonds and includes 4-70 amino acid residues, preferably 4-40 amino acid residues, more preferably 4-30 amino acid residues, and most preferably 4-20 amino acid residues.

The polypeptide represented by the above Formula I refers to the consensus sequence (Cys-Xaa-Xaa-Cys-Xaa-Xaa-Xaa-Xaa-His-Xaa-Xaa-Xaa-Xaa-Cys) of the zinc finger domain present in the NC peptide of various retroviruses (for example, human immunodeficiency virus (HIV), murine leukemia virus (MLV), simian immunodeficiency virus (SIV), rous sarcoma virus (RSV), Feline immunodeficiency virus (FIV), and equine immunodeficiency virus (EIV)). The present inventors have confirmed through experiments that the polypeptides including the above sequences are excellent in cell membrane penetration activity and that they can be used as a drug delivery system capable of delivering intracellular substances.

Xaa₁ can be an amino acid selected from the group consisting of Ala, Val, Ile, Leu, Met, Phe, Tyr and Trp as a hydrophobic amino acid and Ser, Thr, Asn and Gln as a polar amino acid. More preferably, the Xaa₁ is an amino acid selected from the group consisting of Phe, Trp, Tyr, Ala and Gln.

Xaa₂ can be an amino acid selected from the group consisting of Ala, Val, Ile, Leu, Met, Phe, Tyr and Trp as a hydrophobic amino acid, Arg, His and Lys as a basic amino acid and Ser, Thr, Asn and Gln as a polar amino acid. More preferably, the Xaa2 is an amino acid selected from the group consisting of Asn, Thr, Lys, Leu and Tyr.

Further, the Xaa₃ and Xaa₄ can individually be an amino acid selected from the group consisting of Asp, Glu, Arg, His, Lys, Ser, Thr, Asn, Gln and Gly as a hydrophilic amino acid. More preferably, the Xaa₃ is an amino acid selected from the group consisting of Gly, Asp and Lys. More preferably, the Xaa₄ is an amino acid selected from the group consisting of Arg, Ser, Gly and Glu.

Further, the Xaa₅ can be an amino acid selected from the group consisting of Asp, Glu, Arg, His, Lys, Ala, Val, Ile, Leu, Met, Phe, Tyr, Trp and Pro except for the polar amino acid. More preferably, the Xaa₅ is an amino acid selected from the group consisting of Pro, Glu, Lys, Ile and Met.

Further, the Xaa₆ and Xaa₇ can be individually an amino acid selected from the group consisting Ala, Val, Ile, Leu, Met, Phe, Tyr and Trp as a hydrophobic amino acid and Ser, Thr, Asn and Gln as a polar amino acid. More preferably, the Xaa6 is an amino acid selected from the group consisting of Thr, Asn, Gln, Met, Tyr and Trp. More preferably, the Xaa7 is an amino acid selected from the group consisting of Ala, Met and Gln.

Further, the Xaa₈ can be an amino acid of Lys, Ala or Arg. The Xaa9 is an amino acid selected from the group consisting of Asp and Glu as an acidic amino acid and Ser, Thr, Asn and Gln as a polar amino acid. More preferably, the Xaa9 is Asp, Glu, Asn or Gln.

The peptide including the amino acid sequence represented by the Formula I can be a polypeptide including a zinc finger domain of a retrovirus, in which the polypeptide may be selected from the group consisting of polypeptide having the amino acid sequences represented by SEQ ID NO: 17 to SEQ ID NO: 23.

Another aspect of the present document relates to an intracellular delivery carrier in which a cargo of an object to be delivered into a cell is conjugated with the end of an NC peptide of a retrovirus.

As used herein, the term "NC peptide" refers to a polypeptide that constitutes the nucleocapsid of a retrovirus. It is known that the polypeptide binds strongly to the retroviral genomic RNA to form the ribonucleoprotein core complex.

As used herein, the term "cell penetrating peptide (CPP)" refers to a peptide having the ability to carry a cargo of an object to be delivered into a cell *in vitro* and/or *in vivo.* The invention is limited to in vitro delivery. The cell penetrating peptide means a peptide having an amino acid sequence that can pass through the cell membrane of a phospholipid bilayer itself.

The retrovirus may be selected from the group consisting of human immunodeficiency virus (HIV), murine leukemia virus (MLV), simian immunodeficiency virus (SIV) and rous sarcoma virus (RSV) and may preferably be HIV.

As used herein, the term "cargo" refers to a chemical substance, a small molecule, a polypeptide, a nucleic acid and the like, which can be conjugated with NC peptides that act as a cell membrane penetrating peptide to be carried into cells.

In the present invention, the substance that can be conjugated with the end of the NC peptide according to SEQ ID NO: 12-15 of retrovirus or the peptide represented by any one ofSEQ ID NO: 17-23, that is, a substance that can be a cargo, is selected from a drug, a contrast agent (e.g., T1 contrast agent, T2 contrast agent such as a superparamagnetic substance, a radioisotope, etc.), a fluorescent marker, a dyeing agent, a polypeptide selected from proteins that are involved in cell immortalization (e.g., SV40 large T antigen and telomerase), anti-apoptotic proteins (e.g., mutant p53 and BclxL), antibodies, cancer genes (e.g., ras, myc, HPV E6/E7 and adenoviridae Ela), cell cycle regulating proteins (e.g., cyclin and cyclin-dependent phosphorylase) and enzymes (e.g., green fluorescent protein, beta-galactosidase and chloramphenicol acetyltransferase, a nucleic acid selected from RNA, DNA or cDNA, antisense oligonucleotides and siRNA, a carbohydrate and a lipid. Nucleotides as nucleic acid cargo may be standard nucleotides (e.g., adenosine, cytosine, guanine, thymine, inosine and uracil) or analogs (e.g., phosphorothioate nucleotides). For example, the nucleic acid cargo may be an antisense sequence consisting of a phosphorothioate nucleotide or RNAi.

According to an embodiment of the present invention, the substance conjugated with the NC peptide having an amino acid sequence represented by SEQ ID NO: 12-15 or the peptide represented by the SEQ ID Nos: 17-23 may penetrate the cell membrane with very high efficiency and remain in the cytoplasm and the nucleus in the cell.

The NC peptide may have one or more zinc finger domains and preferably two zinc finger domains. According to the invention, the NC peptide is selected from the group consisting of polypeptides having the amino acid sequences represented by SEQ ID NO: 12 to SEQ ID NO: 15.

Another aspect of the present invention provides an in vitro method as defined in claims 4-6 for delivering a cargo of an object to be delivered into a cell, the method including contacting the intracellular delivery carrier in which a cargo of an object to be delivered into a cell is conjugated with the end of a peptide including an amino acid sequence represented by the SEQ ID Nos: 17-23 or an intracellular delivery carrier in which a cargo of an object to be delivered into a cell is conjugated with the end of an NC peptide of a retrovirus having the amino acid sequence represented by SEQ ID Nos: 12-15 with a cell.

Since the methods of the present invention utilize intracellular delivery carriers, the description common to both is omitted in order to avoid the excessive complexity of the present application.

Meanwhile, when the peptide containing the amino acid sequence represented by the SEQ ID Nos: 17-23 to which a cargo is conjugated or the NC peptide having the amino acid sequence represented by SEQ ID Nos: 12-15 to which a cargo is conjugated contacts the cell membrane *in vitro,* the cargoes conjugated with peptides are delivered into the cell. There is no particular requirement for the contact between the intracellular delivery carrier and the cell membrane, for example, a limited time, temperature and concentration. It can be carried out under the general conditions applicable to cell membrane penetration in the art.

### [Advantageous Effects]

The intracellular delivery carrier including the peptide including the amino acid sequence represented by the SEQ ID Nos: 17-23 of the present invention or the intracellular delivery carrier including the NC peptide having the amino acid sequence represented by SEQ ID Nos: 12-15 has an advantage of efficiently transferring substances into cells even at a low concentration thereof compared with the existing peptide derived from the virus.

### [Brief Description of Drawings]

FIG. 1 shows the results of confirming the expression of NC-EGFP protein by SDS-PAGE.
FIG. 2 shows peptide sequences of several retroviruses and the results of comparing the cell membrane penetration activity of several NC peptides conjugated with FITC, in which the red box represents the zinc finger domain.
FIGS. 3A to 3C show the results of confirming the cell membrane penetration activity of FITC-HIV-NC peptide (nucleocapsid peptide) in RAW264.7 cells in a concentration-dependent manner or time-dependent manner.
FIG. 4 shows the result of confirming the cell membrane penetration activity of the HIV-NC peptide by immunostaining.
FIGS. 5A to 5E show the results of confirming the cell membrane penetration activity of FITC-HIV-NC peptides in RAW264.7 (FIG. 5A), THP-1 (FIG. 5B), CEM (FIG. 5C), MDCK (FIG. 5D) and 293FT (FIG. 5E) in a concentration-dependent manner.
FIGS. 6A to 6C show the results of comparing cell membrane penetration activity of GFP-HIV-NC peptide and GFP-Tat peptide in RAW264.7 cells in a concentration-dependent manner.
FIG. 7 shows the results of comparing cell membrane penetration activity of FITC-HIV-NC peptide, FITC-Tat peptide, FITC-MA11 peptide, FITC-PTD-ys peptide, FITC-TLM peptide and FITC-TD1 peptide.
FIG. 8 shows the result of confirming the degree of cell membrane penetration activity of FITC-13NC35 peptide.
FIG. 9 shows the results of confirming the degree of cell membrane penetration activity of FITC-29NC50 peptide.
FIG. 10 shows the results of confirming whether the distribution of HIV-NC peptides in tissue cells after the penetration into tissues *in vivo* in which the scale bar represents 50 µm in each photograph(not within the scope of the invention).
FIG. 11 shows the results of confirming the distribution of HIV-NC peptides in retinal cells after the penetration into ocular tissue: GCL means ganglion cell layer, INL means inner nuclear layer, and ONL means outer nuclear layer.
FIG. 12 shows the results of exhibiting cell membrane penetration activity of hexapeptide conjugated with HIV-NC peptide.
FIG. 13 shows the results of exhibiting the cell membrane penetration activity of RNA conjugated with HIV-NC peptide.

### [Mode for Invention]

Hereinafter, one or more embodiments and aspects are described in more detail by way of Examples. The scope of the present invention defined by the appended claims is not limited to these Examples.

### Experimental method

### 1. Construction of a recombinant vector for NC peptide expression

A recombinant vector was prepared as follows to confirm the expression of a recombinant protein conjugated with NC peptide and EGFP and to purify the protein. Polymerase chain reaction (PCR) was performed using a primer including restriction enzyme recognition sequences in order to add restriction enzyme recognition sequences at the N-terminus of the NC peptide gene to allow NdeI which is a restriction enzyme (New England Biolabs; NEB, USA) to act on and at the C-terminus of the NC peptide gene to allow BspEI to act on. The primer sequences (SEQ ID NO: 1 and SEQ ID NO: 2) used in PCR and the PCR conditions, respectively, are shown in Tables 1 and 2 below.

**[Table 1]**

| | SEQ. ID NO. | Sequence |
|---|---|---|
| NC-NdeI | SEQ. ID NO. 1 | CATATGCAGCGGGGAACTT |
| NC-BspEI | SEQ. ID NO. 2 | TCCGGAGTTTGCCTGTCTC |

**[Table 2]**

| PCR reactants | | PCR cycle | | |
|---|---|---|---|---|
| dH₂O | 37.5 µl | 95°C | 2 minutes | |
| dNTP (10X) | 4 µl | 95°C | 1 minute | 30 times |
| F primer (10 µM) | 1 µl | 65°C | 30 seconds | |
| R primer (10 µM) | 1 µl | 74°C | 4 minutes | |
| NC peptide gene | 1 µl | 74°C | 5 minutes | |
| DNA Taq polymerase | 0.5 µl | 4°C | Unlimited | |
| buffer | 5 µl | | | |
| total | 50 µl | | | |

Next, in order to add restriction enzyme recognition sequences at the N-terminus of enhanced green fluorescent protein (EGFP) gene to allow BspEI to act on and at the C-terminus of the EGFP gene to allow HindIII to act on, PCR was carried out under the same conditions as in Table 2 as described above. However, the primers of SEQ ID NO: 3 and SEQ ID NO: 4 described in Table 3 below were used as the primers.

**[Table 3]**

| | SEQ. ID NO. | Sequence |
|---|---|---|
| EGFP-BspEI-F | SEQ. ID NO. 3 | TCCGGAGTGAGCAAGGGCGA |
| EGFP-HindIII-R | SEQ. ID NO. 4 | AAGCTTCTTGTACACTCTCGT |

The NC peptide gene and the EGFP gene to which the restriction enzyme recognition sequence was added were reacted at 16°C for 12 hours, resulting in the ligation. PCR was performed on the ligated NC peptide-EGFP gene (hereinafter referred to as 'NC-EGFP gene') (using primers of SEQ ID NOS: 1, 2, 3 and 4) to obtain the entire sequence of NC-EFGP gene. The conditions of NC-EGFP gene ligation reaction are shown in Table 4 below.

**[Table 4]**

| | |
|---|---|
| dH₂O | 6 µl |
| T4 DNA Ligase buffer (10X) | 1 µl |
| NC peptide DNA (50 ng/µℓ) | 1 µl |
| EGFP gene DNA (50 ng/µℓ) | 1 µl |
| T4 DNA Ligase (400 units/µl) | 1 µl |
| Total | 10 µl |

Then, pET21a (Novagen, USA) vector was cut with NdeI and HindIII restriction enzymes. Then, vector fragments were isolated according to the manufacturer's protocol using a PCR purification kit (Qiagen, USA). The restriction enzyme reaction was performed using NEBuffer #2 at 37°C for 2 hours. The isolated pET21a vector fragment was ligated with the NC-EGFP gene, and then the recombination vector was isolated using the PCR Purification Kit. The recombinant vector into which the NC-EGFP gene was inserted was named pET21a NC-EGFP.

In order to replicate the pET21a NC-EGFP vector, the vector was transformed into *E. coli* DH5α and shake-cultured at 37°C until the OD was 0.5 to 0.6 in LB liquid medium. After the culture was completed, the culture solution was centrifuged to collect the *E. coli* pellet, and the pET21a NC-EGFP vector was isolated from the *E. coli* pellet collected according to the manufacturer's protocol using a plasmid extraction kit (Qiagen). The isolated pET21a NC-EGFP vector was identified by quantifying the concentration using the UV method.

### 2. Expression, isolation and purification of NC-EGFP

In order to confirm the protein expression of the pET21a NC-EGFP vector prepared as described above, the following experiment was conducted.

PET21a NC-EGFP vector was transformed into BL21 (DE3) (Thermo Fisher, USA), plated on LB plate and cultured at 37°C for 12 hours. Colonies formed after 12 hours were inoculated into LB liquid medium and further cultured at 37°C. After about 12 hours, the culture solution having reached an OD of 0.5 to 0.6 was inoculated into 250 ml of LB liquid medium and cultured at 37°C for 3 hours to 4 hours to have an OD of 0.5 to 0.6. When the OD of the culture solution reached 0.5 to 0.6, 0.5 mM isopropyl β-D-thiogalactoside (IPTG) was added to the culture solution. Then, the culture was performed at 25°C for 24 hours. After 24 hours, the culture solution was centrifuged to obtain BL21 pellet. The obtained pellet was suspended in a dissolution buffer (50 mM NaH₂PO₄, 300 mM NaCl, 10 mM imidazole and pH 8.0), and then the *E. coli* was disrupted (amplitude 40%) using an ultrasonic wave crusher.

The *E. coli* debris was centrifuged and separated into supernatant and precipitate, and the supernatant was filled into a tube using a 0.45 µm filter. The supernatant filled in the tube was placed in a column packed with Ni-NTA (nitrilotriacetic acid) resin to bind the protein and the resin to each other. In order to remove foreign proteins that did not bind to the resin, the resin was then washed with wash buffer (50 mM NaH₂PO₄, 300 mM NaCl, 20 mM imidazole and pH 8.0). The final protein was obtained in a gradient mobile phase using an imidazole-added buffer (50 mM NaH₂PO₄, 300 mM NaCl, 250 mM imidazole and pH 8.0). Next, in order to remove imidazole, the obtained protein was placed in a membrane tube, and buffer exchange by osmotic action was performed using a buffer (20 mM NaH₂PO₄, 300 mM NaCl and pH 7.2) not containing imidazole. Finally, the concentration of the protein dissolved in the buffer not containing imidazole was measured by Bradford assay to identify NC-EGFP protein expression. The results confirmed that even if other sequences were conjugated with the NC gene sequence, it did not affect the NC peptide expression.

FIG. 1 shows the results of the NC-EGFP protein by SDS-PAGE, which shows that the NC-EGFP protein having a size of about 35 kDa was expressed.

### 3. Analysis of cell membrane penetration activity of NC peptides

### 3-1. Cell culture

HeLa, RAW 264.7 and 293 FT cells were cultured in DMEM medium supplemented with 10% FBS and 100 U/ml penicillin/streptomycin. MDCK cells were cultured in EMEM medium supplemented with 10% FBS and 100 U/ml penicillin/streptomycin (Hyclone, Logan, UT, USA). THP-1 and CEM cells were cultured in RPMI-1640 medium supplemented with 10% FBS and 100 U/ml penicillin/streptomycin (Hyclone, Logan, UT, USA). All cells were cultured in a humidified thermostat in 5% CO₂ at 37C.

### 3-2. Analysis of cell membrane penetration activity of NC peptide - Fluorescence-activated cell sorting (FACS)

Various types of cells cultured in the Example 3-1 as described above were seeded into 6-well plates at a density of 1 × 10⁶ cells/well, and cultured for 24 hours to attach the cells to the plates. Then, cells were treated with the FITC-conjugated NC peptide (hereinafter referred to as "FITC-NC peptide"), the EGFP-conjugated NC peptide (hereinafter referred to as "NC-EGFP peptide") and the EGFP-conjugated Tat peptide (hereinafter referred to as "Tat-EGFP peptide"), respectively, at different peptide concentrations (0.5, 1.0, 2.5, 5.0 and 10 µM) or different peptide treatment time. The peptides were prepared by chempeptide (China) and Life tein (USA). The cells were then washed three times with PBS, and the cells were separated with trypsin-EDTA and suspended in PBS containing 2% FBS/0.1% bovine serum albumin (BSA). The suspension was centrifuged at 2,000 rpm for 2 minutes to obtain cell pellets, and the cells obtained were fixed with 3.7% formaldehyde for 20 minutes. Then, cell pellets were obtained by centrifugation at 2,000 rpm for 2 minutes, washed twice with PBS and then suspended in PBS containing 2% FBS and 0.1% BSA. Cells were analyzed by flow cytometry using a fluorescence activated cell sorter (FACS Calibur, Beckon Dickinson, CA, USA).

### 3-3. Analysis of cell membrane penetration activity of NC Peptide - Immunostaining

HeLa cells were seeded into a 12-well plate having glass at a density of 1 × 10⁵ cells/well, and then cultured for 24 hours to attach the cells to the glass. Then, cells were treated with FITC-NC peptides at 3 mM concentration for 3 hours. After 3 hours, the cells were washed three times with PBS, the cells were fixed with 3.7% formaldehyde for 20 minutes, and the cells were treated with PBS containing 0.2% Triton X-100 to increase the cell membrane penetration activity. Then, the cells were blocked with 3% BSA for 1 hour, reacted with lamin A/C antibody (Sigma-Aldrich, USA) at room temperature for 2 hours, and washed three times with PBS. Next, the cells were treated with Cy3-conjugated secondary antibody (Jackson ImmunoResearch, USA) at room temperature for 1 hour, washed twice with PBS, and then stained with DAPI (4',6-diamidino-2-phenylindol) for 10 minutes. The HeLa cell-attached glass was removed and placed on a slide glass. Then, the cells were observed with a confocal laser scanning microscope (LSM 700, Zeiss, Germany).

In addition, the following method was used to compare the penetration activity of NC peptides with other cell penetrating peptides.

The HeLa cells were seeded into a 12-well plate containing glass at a density of 1 × 10⁵ cells/well, and then cultured for 24 hours to attach the cells to the glass. Thereafter, the FITC-NC peptide, the trans-activating transcriptional activator peptide (FITC-Tat peptide), the FITC-MA11 peptide, the protein transduction domain-ys peptide (FITC-PTD-ys peptide), the translocation motif peptide (FITC-TLM peptide) and FITC-TD1 peptide were treated with the HeLa cells for 3 hours. After 3 hours, the cells were washed 3 times with PBS and the cells were fixed with 3.7% formaldehyde for 20 minutes. The cells were treated with PBS containing 0.2% Triton X-100 to increase cell membrane penetration activity and blocked with 3% BSA for 1 hour. Then, the cells were reacted with tubulin antibody (Santa Cruz Biotechnology, USA) at room temperature for 2 hours and washed three times with PBS. The cells were treated with Cy3 secondary antibody. They were reacted at room temperature for 1 hour, washed twice with PBS, and stained with DAPI for 10 minutes. The HeLa cell-attached glass was removed and placed on a slide glass. Then, the cells were observed with a confocal laser scanning microscope.

Further, in order to identify a change in cell membrane penetration activity by FITC-NC peptide, the HeLa cells were treated with hexapeptide, FITC-hexapeptide, FITC-NC-hexapeptide, FITC-13NC35 peptide and FITC-29NC50 peptide for 3 hours in the same manner as above and observed with a confocal laser scanning microscope. Meanwhile, the amino acid sequences of the hexapeptide, PTD-ys peptide, TLM peptide, TD1 peptide, NC-hexapeptide, 13NC35 peptide and 29NC50 peptide used are shown in Table 5 below.

**[Table 5]**

| Name | SEQ. ID NO. | Amino acid Sequence |
|---|---|---|
| Hexa peptide | SEQ. ID NO. 5 | EEMQRR |
| PTD-ys peptide | SEQ. ID NO. 6 | YARVRRRGPRR |
| TLM peptide | SEQ. ID NO. 7 | PLSSIFSRIGDP |
| TD1 peptide | SEQ. ID NO. 8 | KAMININKFLNQC |
| NC-hexa peptide | SEQ. ID NO. 9 | VKCFNCGKEGHTARNCRAPRKKGCW KCGKEGHQMKDCTEEEMQRR |
| 13NC35 peptide | SEQ. ID NO. 10 | VKCFNCGKEGHTARNCRAPRKKG |
| 29NC50 peptide | SEQ. ID NO. 11 | RAPRKKGCWKCGKEGHQMKDCT |

### 3-4. Analysis of cell membrane penetration activity of NC Peptide - Fluorometer

RAW264.7 cells were seeded into 24-well plates at a density of 5 × 10⁴ cells/well and cultured for 24 hours to attach the cells to the plates. Then, each of the Tat-EGFP peptide and the NC-EGFP peptide was treated at different concentrations for 1 hour. After 1 hour, the cells were washed three times with PBS, and 100 ml of radioimmunoprecipitation assay (RIPA) buffer was added to dissolve the cells at 4°C for 30 minutes. 100 ml of the cell lysate was transferred to a 96-well plate for fluorescence analysis, and GFP fluorescence was measured using a fluorescence analyzer (Synergy MX, BIOTEK, USA).

Further, NC peptides of FITC-conjugated Human immunodeficiency virus (HIV), Murine leukemia virus (MLV), Simian immunodeficiency virus (SIV) and RSV (Rous sarcoma virus) (hereinafter referred to as FITC-HIV-NC, FITC-MLV-NC, FITC-SIV-NC and FITC-RSV-NC, respectively) and Tat peptide were treated with RAW264.7 cells at a concentration of 1.0 µM for 1 hour. Then, the fluorescence signal of the cells was measured.

### 3-5. Change in RNA transduction efficiency by NC Peptide

MT4 cells were seeded into 24-well plates at a density of 2 × 10⁵ cells/well and cultured in a humidified thermostat in 5% CO₂ at 37°C for 24 hours. 2 µl of NC peptides and 40 nM siGLO (Green transfection indicator, Dharmacon, D-001630-01-05) having different concentrations were mixed and reacted at room temperature for 30 minutes and then treated to MT4 cells. After further culture for 24 hours, MT4 cells were washed three times with PBS and analyzed by flow cytometry using a fluorescence activated cell sorter. Further, the cells were observed with a fluorescence inverted microscope (Olympus).

### 4. In vivo Tissue penetration of NC peptide and distribution of NC peptide in tissue cells (not within the scope of the invention)

The FITC-HIV-NC peptide and FITC-Tat peptide having a concentration of 3.2 mg/100 µl, respectively, were injected into mouse tail vein using a 30 gauge syringe. The kidney, liver, lung, heart and spleen of the mice were removed 2 hours after the injection of the peptide and fixed with 4% paraformaldehyde for 1 hour. They were placed in 30% sucrose until the tissue was subsided. Then, each tissue was frozen using an OCT compound (Leica, Germany), and a tissue section slide was prepared with a freezing sectional device. Tissue section slides were stained with DAPI for 10 minutes according to methods known in the art and observed with a confocal laser scanning microscope.

In addition, 1 µl of a 100 µM stock of FITC-HIV-NC peptide was intravitreally injected, and 2 µl of a 100 µM stock of FITC-HIV-NC peptide was subretinally injected. After 24 hours, mouse eye tissue was extracted. Eye tissue section slides were prepared in the same manner as described above. Slides were stained with DAPI for 10 min and observed with a confocal laser scanning microscope.

### Experiment result

### 1. Identification of cell membrane penetration activity of various retroviral NC peptides

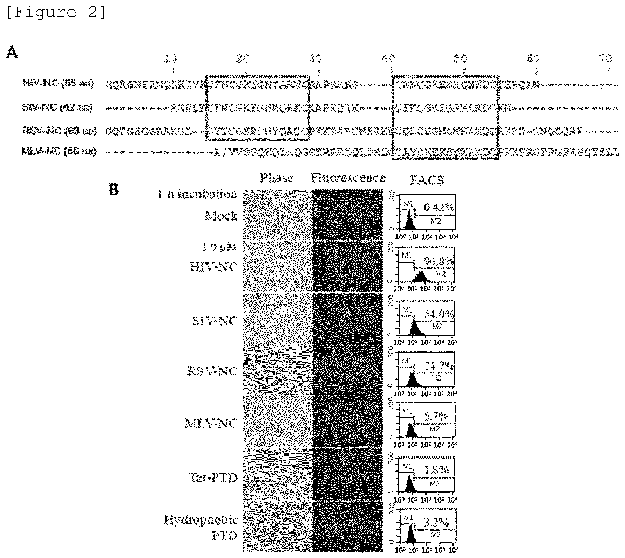

In order to confirm whether the degree of cell membrane penetration activity was varied depending on the degree of similarity of NC peptide sequence, RAW264.7 Cells were treated with 1.0 µM FITC-conjugated NC peptide of HIV, SIV, RSV and MLV and Tat peptide for 1 hour. As a result, as shown in FIG. 2, it was confirmed that NC peptides of HIV, SIV, RSV and MLV have excellent cell membrane penetration activity through the FITC fluorescence brightness. FACS analysis was performed to indicate that NC peptides of HIV, SIV, RSV and MLV, respectively, showed fluorescence in 96.8%, 54.0%, 24.2% and 5.7% of cells. The results of this experiment indicate that NC peptides of several retroviruses including HIV have excellent cell membrane penetration activity compared with that of the conventional Tat peptides. As shown in FIG. 2, it was confirmed that the NC peptides of HIV, SIV, RSV and MLV contained a zinc finger domain as a consensus sequence.

Meanwhile, the amino acid sequences of the NC peptides of HIV, SIV, RSV and MLV used above are shown in Table 6 below.

**[Table 6]**

| Name | SEQ. ID NO. | Amino acid Sequence |
|---|---|---|
| HIV-NC peptide | SEQ. ID NO. 12 | MQRGNFRNQRKIVKCFNCGKEGHTARNCRAPRKKG CWKCGKEGHQMKDCTERQAN |
| SIV-NC peptide | SEQ. ID NO. 13 | RGPLKCFNCGKFGHMQRECKAPRQIKCFKCGKIGH MAKDCKN |
| RSV-NC peptide | SEQ. ID NO. 14 | GQTGSGGRARGLCYTCGSPGHYQAQCPKKRKSGNS RERCQLCDGMGHNAKQCRKRDGNQGQRP |
| MLV-NC peptide | SEQ. ID NO. 15 | ATVVSGQKQDRQGGERRRSQLDRDQCAYCKEKGHW AKDCPKKPRGPRGPRPQTSLL |

### 2. Identification of cell membrane penetration activity of HIV-NC peptide

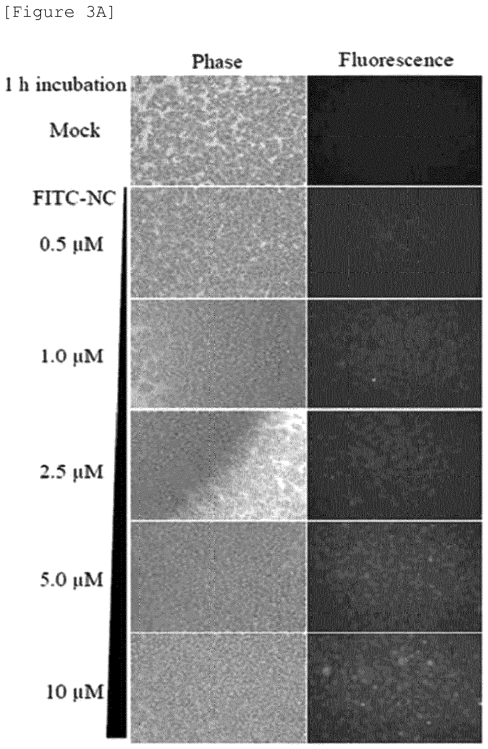

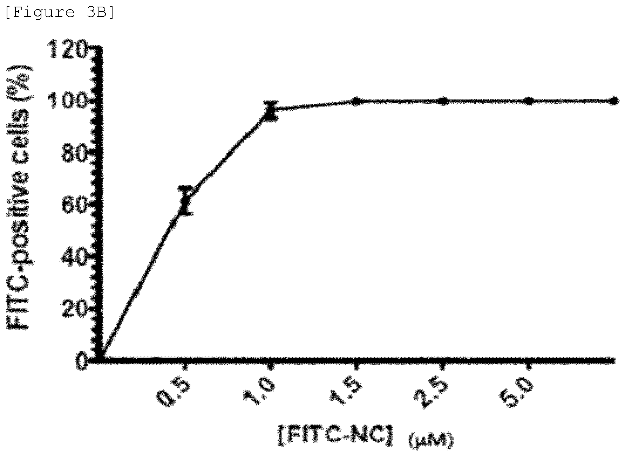

In order to examine the cell membrane penetration activity of HIV-NC peptide (SEQ ID NO: 12) having the highest cell membrane penetration activity in the above experiment according to its concentrations, RAW264.7 macrophages were treated with FITC-HIV-NC peptide at a concentration of 0.5, 1, 2.5, 5.0 and 10 µM for 1 hour. The fluorescence intensity of the cells was measured using a fluorescence microscope (Leica Micoscope Systems, Germany). As shown in FIG. 3A, it was confirmed that as the concentration of FITC-HIV-NC peptide increases, the penetration activity of the peptide increases. Further, as a result of the fluorescence analysis, as shown in FIG. 3B, it was confirmed that as the concentration of FITC-HIV-NC peptide was increased, the fluorescence signal was increased, indicating that the cell membrane penetration activity was increased.

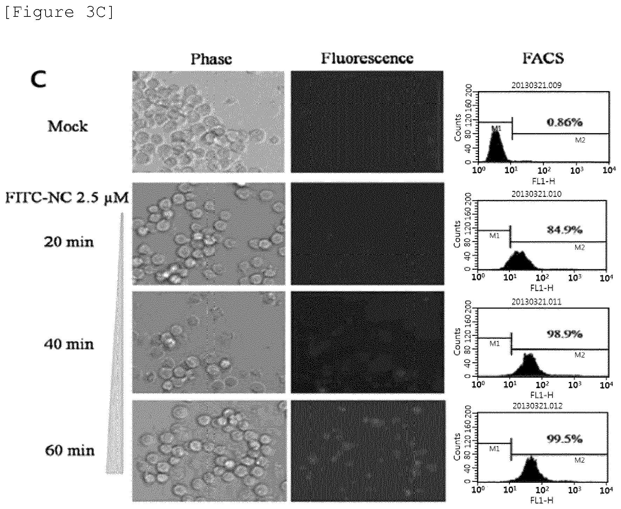

Further, in order to confirm the degree of cell membrane penetration of FITC-HIV-NC peptides according to the treatment time, the peptides were treated with RAW 264.7 cells at a concentration of 2.5 µm for 20 minutes, 40 minutes and 60 minutes. As a result, it was confirmed by fluorescence microscopy and FACS analysis that as shown in FIG. 3C, the cell membrane permeability of the peptide was significantly increased with increasing time of FITC-HIV-NC peptide treatment.

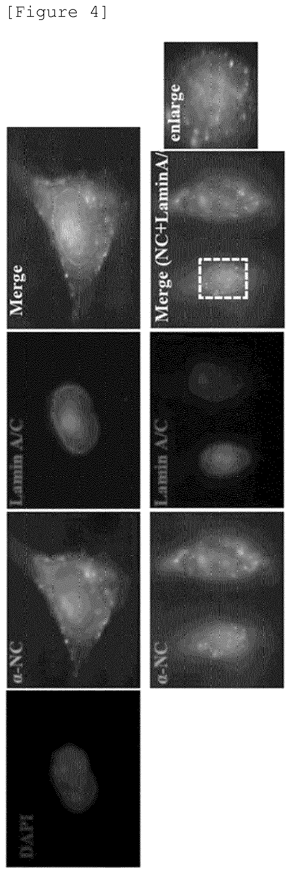

In order to verify the cell membrane penetration activity of HIV-NC peptides, HeLa cells were immunostained and observed with a confocal laser scanning microscope. As a result, it was confirmed that as shown in FIG. 4, many HIV-NC peptides were uniformly distributed in the cell membrane, cytoplasm and nuclear membrane and that some HIV-NC peptides were distributed in the nucleus. The results indicate that HIV-NC peptides can penetrate cell membranes at low concentrations and that HIV-NC peptides penetrate cell membrane quickly.

### 3. Identification of cell membrane penetration activity of HIV-NC peptides in various cells

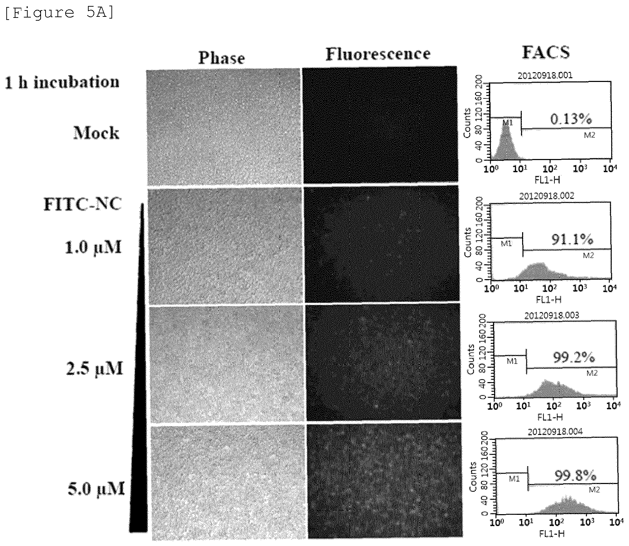

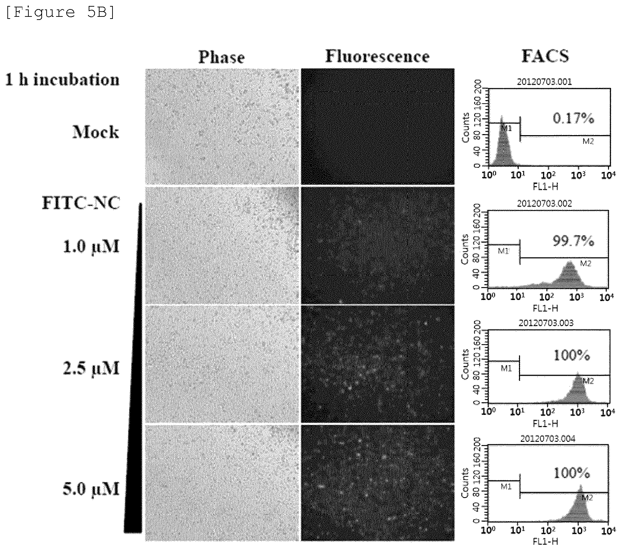

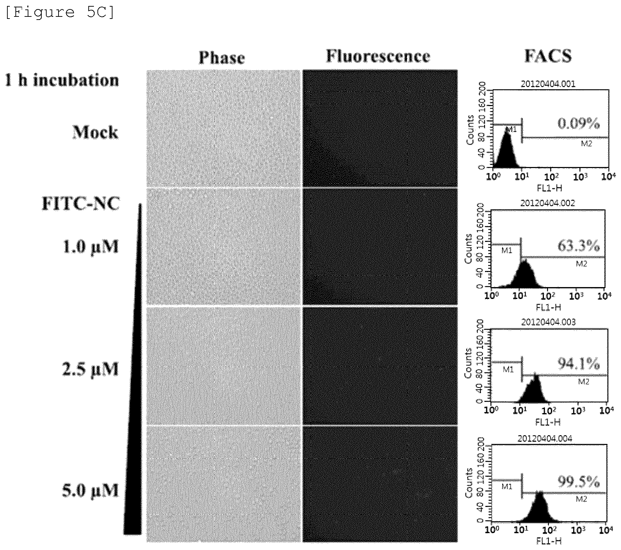

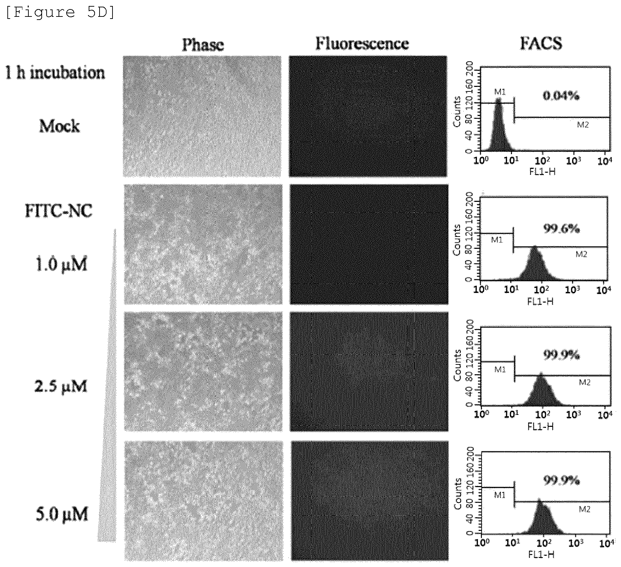

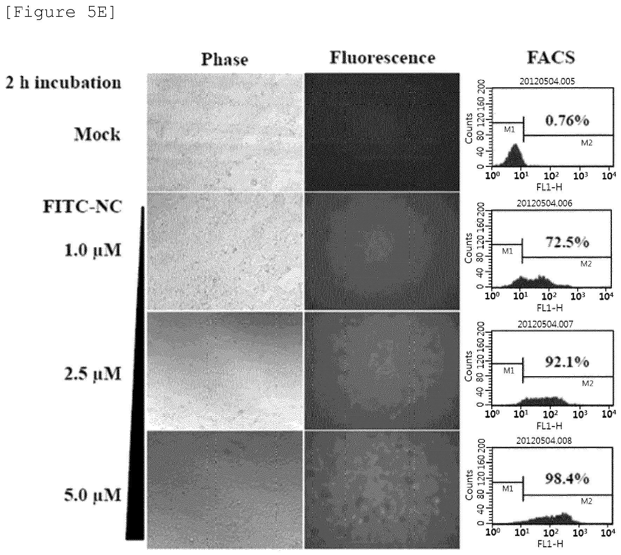

In order to identify whether the cell membrane penetration activity of HIV-NC peptide varies depending on kinds of cells, RAW264.7, THP-1, CEM, MDCK and 293FT cells were treated with FITC-HIV-NC peptide at a concentration of 1.0, 2.5 and 5.0 µM for 1 hour or 2 hours. As a result, it was confirmed by fluorescence microscopy and FACS analysis that as shown in FIGS. 5A to 5E, the cell membrane penetration activity of the peptide was significantly increased as the concentration of FITC-HIV-NC peptide was increased. FIG. 5A shows the results of RAW264.7 (mouse macrophage). FIG. 5B shows the results of THP-1 (human mononuclear cell line) cells, FIG. 5C shows the results of CEM. FIG. 5D shows the results of MDCK (Madin- Darby canine kidney), and FIG. 5E shows the results of 293FT (derived from human fetal kidney cells). The above results indicate that HIV-NC peptides can penetrate various cell membranes of cancer cells, immune cells, epithelial cells and the like.

### 4. Comparison of cell membrane penetration activity between HIV-NC and Tat peptides

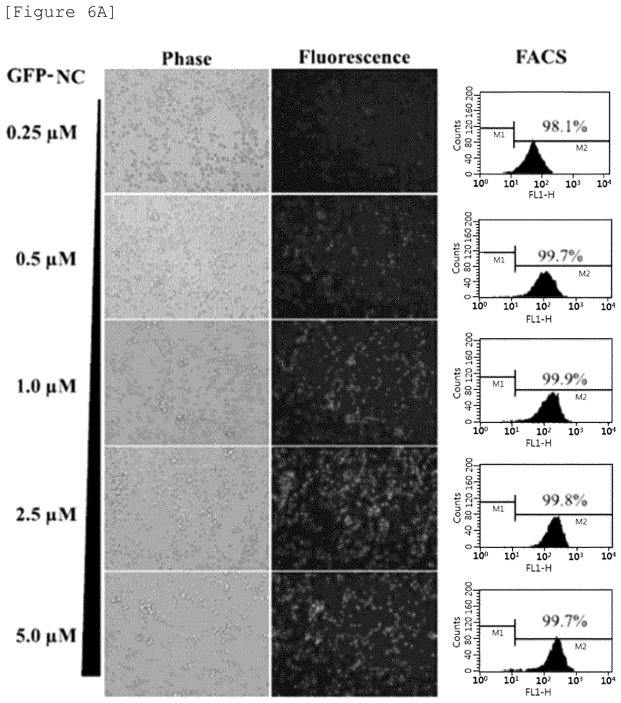

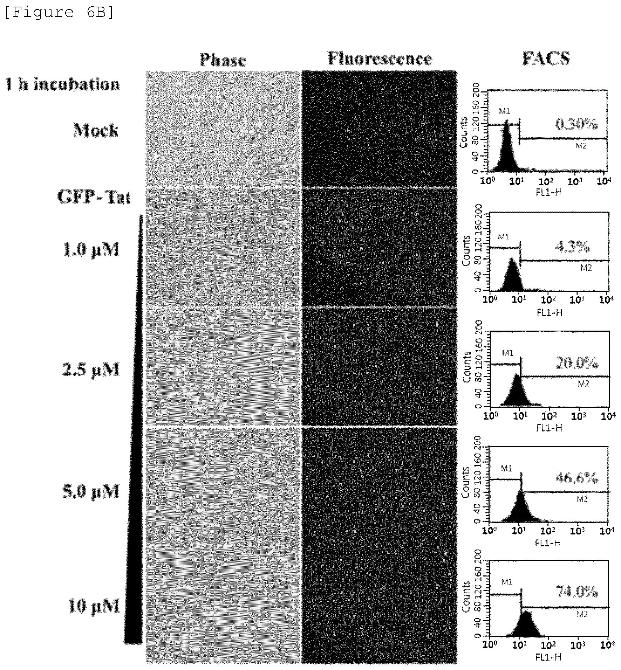

In order to confirm the degree of cell membrane penetration activity of HIV-NC peptides, a comparative experiment carried out with a Tat peptide known as a conventional cell penetrating protein. RAW264.7 cells were treated with green fluorescent protein (GFP)-conjugated HIV-NC peptide (hereinafter referred to as GFP-HIV-NC peptide) at 0.5, 1.0, 2.5 and 5.0 µM and GFP-conjugated Tat peptide at 1.0, 2.5, 5.0 and 10 µM, respectively, for 1 hour. After 1 hour, the fluorescence of the peptides was confirmed. As a result, it was confirmed that as shown in FIGS. 6A and 6B, the GFP-HIV-NC peptide showed fluorescence at a low concentration of 0.25 µM, while the GFP-Tat peptide showed slightly fluorescence at a high concentration of 10 µM. Further, the cells were collected and analyzed by FACS. As a result, it was confirmed that as shown in FIGS. 6A and 6B, GFP-HIV-NC peptides showed fluorescence for 99.9% of cells at a concentration of 1 µM, while GFP-Tat peptides showed fluorescence for 4.3% of cells at the same concentration.

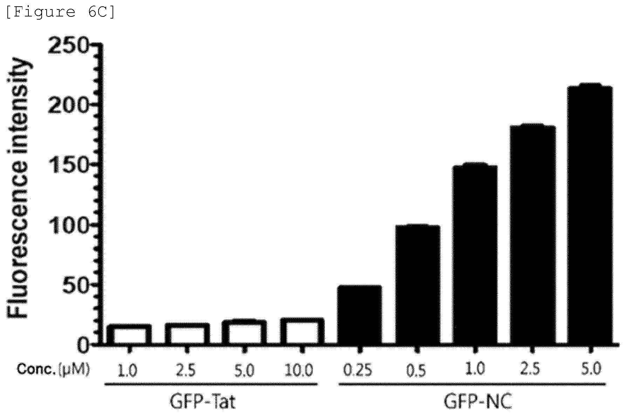

Further, as a result of the fluorescence analysis, it was confirmed that as shown in FIG. 6C, as the concentration of GFP-HIV-NC peptide was increased, the cell membrane penetration activity was also increased, but the GFP-Tat peptide has very low cell membrane penetration activity regardless of the treatment concentration. The results indicate that HIV-NC peptides are highly efficient cell penetrating peptides as compared with Tat peptides, which are well-known as an existing cell penetrating protein.

### 5. Comparison of cell membrane penetration activity of HIV-NC peptides and other cell penetrating peptides

The HeLa cells were treated with FITC-HIV-NC peptides, FITC-Tat peptides, FITC-MA11 peptides, FITC-PTD-ys peptides, FITC-TLM peptides and FITC-TD1 peptides, and then the penetration activity of each peptide was confirmed.

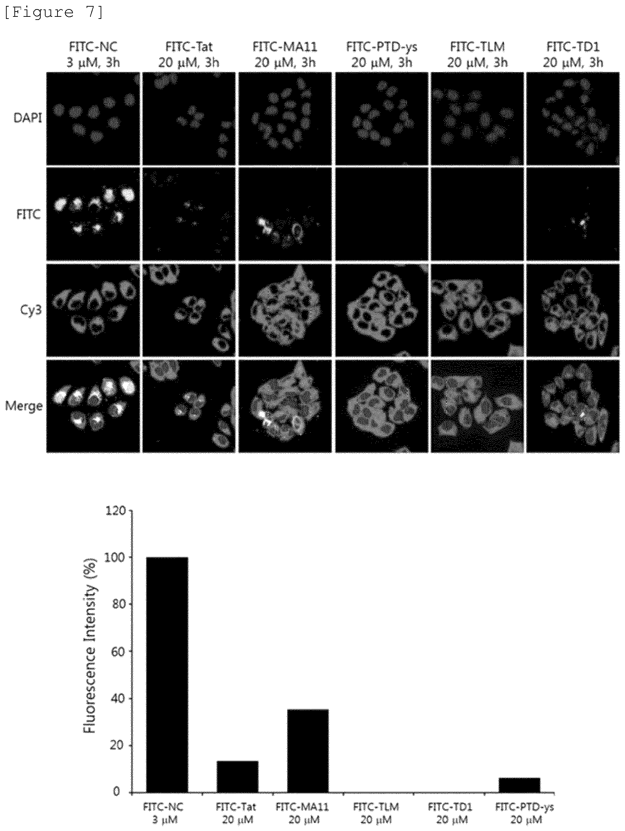

As a result, it was confirmed that as shown in FIG. 7, the FITC-TLM peptides and the FITC-TD1 peptides were hardly penetrable to the cell membrane even though they were treated at a high concentration of 20 µM, but the fluorescence signal was strong and the cell membrane penetration activity was remarkably excellent although FITC-HIV-NC peptides were treated at a low concentration of 3 µM. Further, the cell membrane penetration activity of FITC-HIV-NC peptides is about 2.5 times higher than that of FITC-MA11 peptides, about 7 times higher than that of FITC-Tat peptides and about 10 times higher than that of FITC-PTD-ys peptides.

### 6. Identification of the cell membrane penetration activity of zinc finger domains in NC peptides

Experimental result 1 confirmed that several retroviral NC peptides had cell membrane penetration activity. As shown in FIG. 2, it was confirmed that there are two zinc finger domains in HIV, SIV and RSV, and one zinc finger domain in MLV.

In order to confirm whether the cell membrane penetration activity is caused by the zinc finger domain which is a consensus sequence in several NC peptides, FITC was conjugated with a peptide containing the first zinc finger domain consisting of the 13th amino acid to the 35th amino acid of the HIV-NC peptide (13NC35 peptide, SEQ ID NO: 10), and, FITC was conjugated with a peptide containing the second zinc finger domain consisting of the 29th amino acid to the 50th amino acid of the HIV-NC peptide (29NC50 peptide, SEQ ID NO: 11) so that the cell membrane penetration activity was confirmed in HeLa cells, respectively.

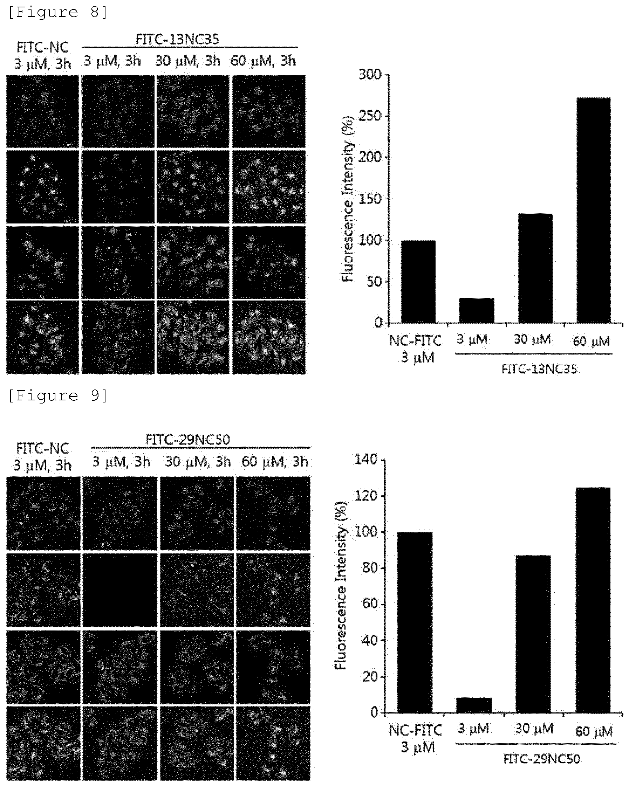

As a result, it was confirmed that as shown in FIGS. 8 and 9, fluorescence signals were increased in proportion to the concentration of both 13NC35 peptide and 29NC50 peptide. This means that the zinc finger domain of the NC peptide is an important part of cell membrane penetration activity.

Meanwhile, the amino acid sequences of the zinc finger domains of NC peptides of HIV, SIV, RSV and MLV are shown in Table 7 below.

**[Table 7]**

| Name | SEQ. ID NO. | Amino acid Sequence |
|---|---|---|
| HIV-NC 1^{st} Zinc finger domain | SEQ. ID NO. 17 | CFNCGKEGHTARNC |
| HIV-NC 2^{nd} Zinc finger domain | SEQ. ID NO. 18 | CWKCGKEGHQMKDC |
| SIV-NC 1^{st} Zinc finger domain | SEQ. ID NO. 19 | CFNCGKEGHMQREC |
| SIV-NC 2^{nd} Zinc finger domain | SEQ. ID NO. 20 | CFKCGKIGHMAKDC |
| RSV-NC 1^{st} Zinc finger domain | SEQ. ID NO. 21 | CYTCGSPGHYQAQC |
| RSV-NC 2^{nd} Zinc finger domain | SEQ. ID NO. 22 | CQLCDGMGHNAKQC |
| MLV-NC Zinc finger domain | SEQ. ID NO. 23 | CAYCKEKGHWAKDC |

### 7. In vivo tissue penetration of HIV-NC peptide and its distribution in tissue cells (not within the scope of the invention)

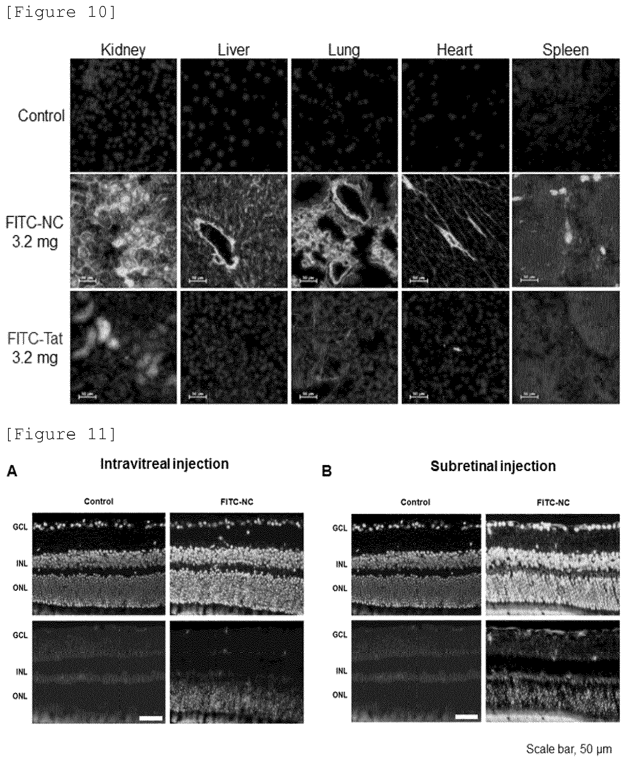

In order to confirm whether the NC peptides penetrate into tissues in vivo and are uniformly distributed in tissue cells, HIV-NC peptides were injected to the mouse tail vein. As a result, it was confirmed that as shown in FIG. 10, the peptides were well distributed in tissue cells of kidney, liver, lung, heart and spleen. In particular, the distribution efficiency to the kidney, liver and lung tissue cells was remarkably excellent. On the other hand, it was confirmed that Tat peptides were hardly distributed in other tissues except for kidney.

Further, HIV-NC peptides were injected through the intravitreal and subretinal injection routes. As a result, it was confirmed that as shown in FIG. 11, the fluorescence signal was strong so that HIV-NC peptides penetrated the eye tissue through the eye injection route to be well distributed in retinal cells.

### 8. Confirmation of cell membrane penetration activity of polypeptide by HIV-NC peptide

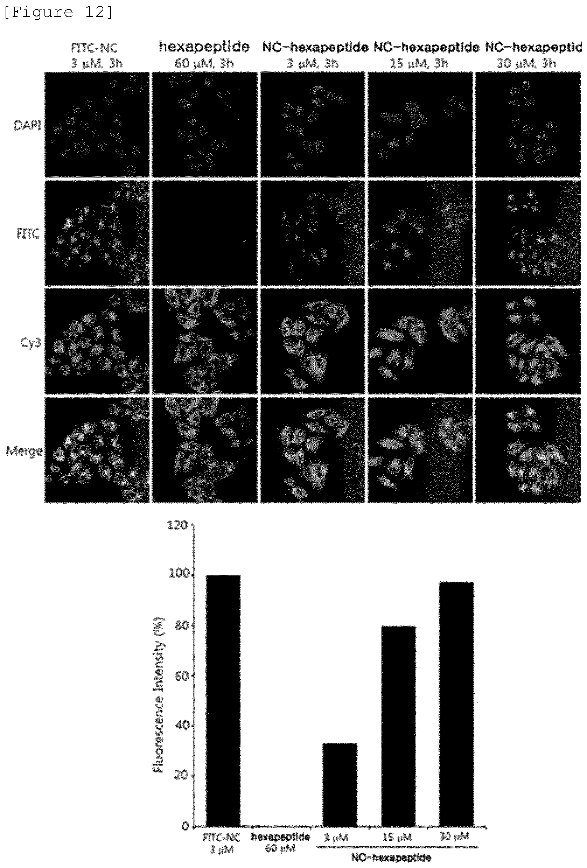

In order to confirm whether HIV-NC peptides using a polypeptide as a cargo can have cell membrane penetration activity, hexapeptide was conjugated to HIV-NC peptide, and cells were treated with the peptides to observe the fluorescence signal. As a result, it was confirmed that as shown in FIG. 12, the hexapeptide itself did not penetrate the cell membrane, but when conjugated with the HIV-NC peptide, they could penetrate cell membrane and that cell membrane penetration activity was increased in proportion to the concentration.

### 9. Confirmation of cell membrane penetration activity of polynucleotide by HIV-NC peptide

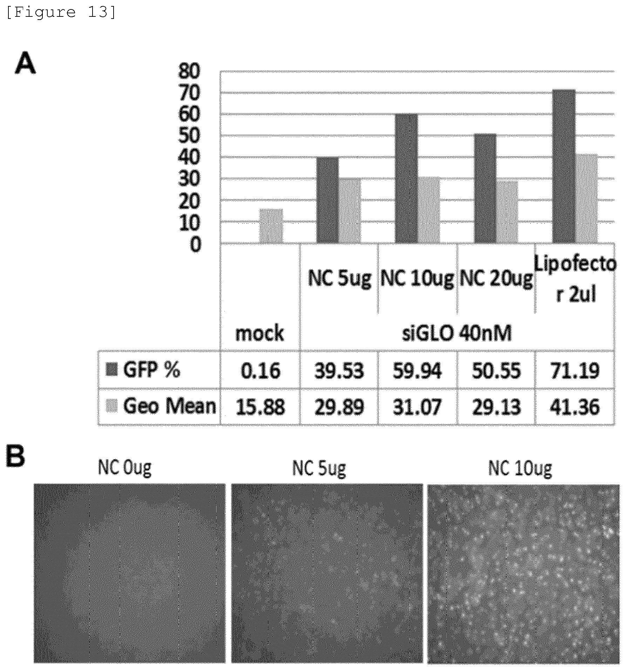

In order to confirm whether HIV-NC peptides using a polynucleotide as a cargo can have cell membrane penetration activity, siGLO RNA was conjugated to HIV-NC peptide, and cells were treated with the peptides to observe the fluorescence signal. As a result, it was confirmed that as shown in FIG. 13, the GFP fluorescence signal was increased according to the treatment concentration of HIV-NC peptide, and the degree of cell membrane penetration activity of siGLO was increased. These results indicate that HIV-NC peptides can significantly improve the cell membrane penetration activity of polynucleotides.

## Claims

1. In vitro use of an intracellular delivery carrier in which a cargo of an object to be delivered into a cell is conjugated with the end of a cell penetrating peptide including an amino acid sequence represented by any one of SEQ ID NOs: 17-23,
wherein the cargo of the object to be delivered into a cell is a chemical substance selected from a drug, a contrast agent (e.g., T1 contrast agent, T2 contrast agent such as a superparamagnetic substance, a radioisotope, etc.), a fluorescent marker, and a dyeing agent, a polypeptide selected from proteins that are involved in cell immortalization (e.g., SV40 large T antigen and telomerase), anti-apoptotic proteins (e.g., mutant p53 and BclxL), antibodies, cancer genes (e.g., ras, myc, HPV E6/E7 and adenoviridae Ela), cell cycle regulating proteins (e.g., cyclin and cyclin-dependent phosphorylase) and enzymes (e.g., green fluorescent protein, beta-galactosidase and chloramphenicol acetyltransferase), a nucleic acid selected from RNA, DNA, cDNA, antisense oligonucleotides and siRNA, a carbohydrate or a lipid,
for delivering the cargo of the object into a cell.

2. In vitro use of an intracellular delivery carrier in which a cargo of an object to be delivered into a cell is conjugated with the end of an NC peptide of a retrovirus wherein the cargo of the object to be delivered into a cell is a chemical substance selected from a drug, a contrast agent (e.g., T1 contrast agent, T2 contrast agent such as a superparamagnetic substance, a radioisotope, etc.), a fluorescent marker, and a dyeing agent, a polypeptide selected from proteins that are involved in cell immortalization (e.g., SV40 large T antigen and telomerase), anti-apoptotic proteins (e.g., mutant p53 and BclxL), antibodies, cancer genes (e.g., ras, myc, HPV E6/E7 and adenoviridae Ela), cell cycle regulating proteins (e.g., cyclin and cyclin-dependent phosphorylase) and enzymes (e.g., green fluorescent protein, beta-galactosidase and chloramphenicol acetyltransferase), a nucleic acid selected from RNA, DNA, cDNA, antisense oligonucleotides and siRNA, a carbohydrate or a lipid, and wherein the NC peptide is selected from the group consisting of polypeptides having the amino acid sequence represented by SEQ ID NO: 12 to SEQ ID NO:15, for delivering the cargo of the object into a cell.

3. The use of claim 2, wherein the retrovirus is selected from the group consisting of human immunodeficiency virus (HIV), murine leukemia virus (MLV), simian immunodeficiency virus (SIV), and RSV (rous sarcoma virus).

4. An *in vitro* method for delivering a cargo of an object to be delivered into a cell,
the method comprising contacting a cell with an intracellular delivery carrier in which the cargo of the object to be delivered into the cell is conjugated with the end of a cell penetrating peptide including an amino acid sequence represented by any one of SEQ ID NOs: 17-23,
wherein the cargo of the object to be delivered into the cell is a chemical substance selected from a drug, a contrast agent (e.g., T1 contrast agent, T2 contrast agent such as a superparamagnetic substance, a radioisotope, etc.), a fluorescent marker, and a dyeing agent, a polypeptide selected from proteins that are involved in cell immortalization (e.g., SV40 large T antigen and telomerase), anti-apoptotic proteins (e.g., mutant p53 and BclxL), antibodies, cancer genes (e.g., ras, myc, HPV E6/E7 and adenoviridae Ela), cell cycle regulating proteins (e.g., cyclin and cyclin-dependent phosphorylase) and enzymes (e.g., green fluorescent protein, beta-galactosidase and chloramphenicol acetyltransferase), a nucleic acid selected from RNA, DNA, cDNA, antisense oligonucleotides and siRNA, a carbohydrate or a lipid.

5. An *in vitro* method for delivering a cargo of an object to be delivered into a cell,
the method comprising contacting an intracellular delivery carrier in which a cargo of an object to be delivered into a cell is conjugated with the end of an NC peptide of a retrovirus with a cell,
wherein the cargo of the object to be delivered into the cell is a chemical substance selected from a drug, a contrast agent (e.g., T1 contrast agent, T2 contrast agent such as a superparamagnetic substance, a radioisotope, etc.), a fluorescent marker, and a dyeing agent, a polypeptide selected from proteins that are involved in cell immortalization (e.g., SV40 large T antigen and telomerase), anti-apoptotic proteins (e.g., mutant p53 and BclxL), antibodies, cancer genes (e.g., ras, myc, HPV E6/E7 and adenoviridae Ela), cell cycle regulating proteins (e.g., cyclin and cyclin-dependent phosphorylase) and enzymes (e.g., green fluorescent protein, beta-galactosidase and chloramphenicol acetyltransferase), a nucleic acid selected from RNA, DNA, cDNA, antisense oligonucleotides and siRNA, a carbohydrate or a lipid, and wherein the NC peptide is selected from the group consisting of polypeptides having the amino acid sequence represented by SEQ ID NO: 12 to SEQ ID NO:15.

6. The method of claim 5, wherein the retrovirus is selected from the group consisting of human immunodeficiency virus (HIV), murine leukemia virus (MLV), simian immunodeficiency virus (SIV), and RSV (rous sarcoma virus)

## Patentansprüche

1. In-vitro-Verwendung eines intrazellulären Verabreichungsträgers, bei dem eine Ladung eines in eine Zelle einzubringenden Objekts mit dem Ende eines zelldurchdringenden Peptids konjugiert ist, das eine Aminosäuresequenz enthält, die durch eine der SEQ ID NOs: 17-23 dargestellt ist,
wobei die Ladung des in eine Zelle einzubringenden Objekts eine chemische Substanz ist, die ausgewählt ist aus einem Arzneimittel, einem Kontrastmittel (z.B. T1-Kontrastmittel, T2-Kontrastmittel wie eine superparamagnetische Substanz, ein Radioisotop usw.), einem Fluoreszenzmarker und einem Färbemittel, einem Polypeptid, das ausgewählt ist aus Proteinen, die an der Zellimmortalisierung beteiligt sind (z.B. SV40 large T-Antigen und Telomerase), anti-apoptotischen Proteinen (z.B. mutiertes p53 und BclxL), Antikörpern, Krebsgenen (z.B. ras, myc, HPV E6/E7 und Adenoviridae Ela), Zellzyklus-regulierenden Proteinen (z.B. Cyclin und cyclinabhängige Phosphorylase) und Enzymen (z.B. grün fluoreszierendes Protein, Beta-Galactosidase und Chloramphenicol-Acetyltransferase), einer Nukleinsäure, ausgewählt aus RNA, DNA, cDNA, Antisense-Oligonukleotiden und siRNA, einem Kohlenhydrat oder einem Lipid,
um die Ladung des Objekts in eine Zelle einzubringen.

2. In-vitro-Verwendung eines intrazellulären Verabreichungsträgers, bei dem eine Ladung eines in eine Zelle einzubringenden Objekts mit dem Ende eines NC-Peptids eines Retrovirus konjugiert ist,
wobei die Ladung des in eine Zelle einzubringenden Objekts eine chemische Substanz ist, die ausgewählt ist aus einem Arzneimittel, einem Kontrastmittel (z.B. T1-Kontrastmittel, T2-Kontrastmittel wie eine superparamagnetische Substanz, ein Radioisotop usw.), einem Fluoreszenzmarker und einem Färbemittel, einem Polypeptid, das ausgewählt ist aus Proteinen, die an der Zellimmortalisierung beteiligt sind (z.B. SV40 large T-Antigen und Telomerase), anti-apoptotischen Proteinen (z.B. mutiertes p53 und BclxL), Antikörpern, Krebsgenen (z.B. ras, myc, HPV E6/E7 und Adenoviridae Ela), Zellzyklus-regulierenden Proteinen (z.B. Cyclin und cyclinabhängige Phosphorylase) und Enzymen (z.B. grün fluoreszierendes Protein, Beta-Galactosidase und Chloramphenicol-Acetyltransferase), einer Nukleinsäure, ausgewählt aus RNA, DNA, cDNA, Antisense-Oligonukleotiden und siRNA, einem Kohlenhydrat oder einem Lipid,
und wobei das NC-Peptid aus der Gruppe ausgewählt ist, die aus Polypeptiden besteht, welche die durch SEQ ID NOs: 12 bis SEQ ID NO:15 dargestellte Aminosäuresequenz aufweisen,
um die Ladung des Objekts in eine Zelle einzubringen.

3. Verwendung nach Anspruch 2, wobei das Retrovirus aus der Gruppe ausgewählt ist, die aus menschlichem Immundefizienz-Virus (HIV), murinem Leukämievirus (MLV), simianem Immundefizienz-Virus (SIV) und RSV (Rous-Sarkom-Virus) besteht.

4. In-vitro-Verfahren zum Einbringen einer Ladung eines in eine Zelle einzubringenden Objekts,
wobei das Verfahren das In-Kontakt-Bringen einer Zelle mit einem intrazellulären Verabreichungsträger umfasst, bei dem die Ladung des in die Zelle einzubringenden Objekts mit dem Ende eines zelldurchdringenden Peptids konjugiert ist, das eine Aminosäuresequenz enthält, die durch eine der SEQ ID NOs: 17-23 dargestellt ist,
wobei die Ladung des in die Zelle einzubringenden Objekts eine chemische Substanz ist, die ausgewählt ist aus einem Arzneimittel, einem Kontrastmittel (z.B. T1-Kontrastmittel, T2-Kontrastmittel wie eine superparamagnetische Substanz, ein Radioisotop usw.), einem Fluoreszenzmarker und einem Färbemittel, einem Polypeptid, das ausgewählt ist aus Proteinen, die an der Zellimmortalisierung beteiligt sind (z.B. SV40 large T-Antigen und Telomerase), anti-apoptotischen Proteinen (z.B. mutiertes p53 und BclxL), Antikörpern, Krebsgenen (z.B. ras, myc, HPV E6/E7 und Adenoviridae Ela), Zellzyklus-regulierenden Proteinen (z.B. Cyclin und cyclinabhängige Phosphorylase) und Enzymen (z.B. grün fluoreszierendes Protein, Beta-Galactosidase und Chloramphenicol-Acetyltransferase), einer Nukleinsäure, ausgewählt aus RNA, DNA, cDNA, Antisense-Oligonukleotiden und siRNA, einem Kohlenhydrat oder einem Lipid.

5. In-vitro-Verfahren zum Einbringen einer Ladung eines in eine Zelle einzubringenden Objekts,
wobei das Verfahren das In-Kontakt-Bringen eines intrazellulären Verabreichungsträger, bei dem die Ladung eines in eine Zelle einzubringenden Objekts mit dem Ende eines NC-Peptids eines Retrovirus konjugiert ist, mit einer Zelle umfasst,
wobei die Ladung des in die Zelle einzubringenden Objekts eine chemische Substanz ist, die ausgewählt ist aus einem Arzneimittel, einem Kontrastmittel (z.B. T1-Kontrastmittel, T2-Kontrastmittel wie eine superparamagnetische Substanz, ein Radioisotop usw.), einem Fluoreszenzmarker und einem Färbemittel, einem Polypeptid, das ausgewählt ist aus Proteinen, die an der Zellimmortalisierung beteiligt sind (z.B. SV40 large T-Antigen und Telomerase), anti-apoptotischen Proteinen (z.B. mutiertes p53 und BclxL), Antikörpern, Krebsgenen (z.B. ras, myc, HPV E6/E7 und Adenoviridae Ela), Zellzyklus-regulierenden Proteinen (z.B. Cyclin und cyclinabhängige Phosphorylase) und Enzymen (z.B. grün fluoreszierendes Protein, Beta-Galactosidase und Chloramphenicol-Acetyltransferase), einer Nukleinsäure, ausgewählt aus RNA, DNA, cDNA, Antisense-Oligonukleotiden und siRNA, einem Kohlenhydrat oder einem Lipid,
und wobei das NC-Peptid aus der Gruppe ausgewählt ist, die aus Polypeptiden besteht, welche die durch SEQ ID NOs: 12 bis SEQ ID NO:15 dargestellte Aminosäuresequenz aufweisen.

6. Verfahren nach Anspruch 5, wobei das Retrovirus aus der Gruppe ausgewählt ist, die aus menschlichem Immundefizienz-Virus (HIV), murinem Leukämievirus (MLV), simianem Immundefizienz-Virus (SIV) und RSV (Rous-Sarkom-Virus) besteht.

## Revendications

1. Utilisation *in vitro* d'un support de délivrance intracellulaire dans lequel une cargaison d'un objet à délivrer dans une cellule est conjuguée à l'extrémité d'un peptide de pénétration cellulaire comprenant une séquence d'acides aminés représentée par l'une quelconque des SEQ ID NO : 17 à 23,
dans laquelle la cargaison de l'objet à délivrer dans une cellule est une substance chimique sélectionnée parmi un médicament, un agent de contraste (par exemple, agent de contraste T1, agent de contraste T2 tel qu'une substance superparamagnétique, un radioisotope, etc.), un marqueur fluorescent, et un agent colorant, un polypeptide sélectionné parmi les protéines qui sont impliquées dans l'immortalisation cellulaire (par exemple, grand antigène T de SV40 et télomérase), les protéines anti-apoptotiques (par exemple, p53 mutante et BclxL), les anticorps, les gènes de cancer (par exemple, ras, myc, HPV E6/E7 et Ela d'adénovirus), les protéines de régulation du cycle cellulaire (par exemple, cycline et phosphorylase dépendante de la cycline) et les enzymes (par exemple, protéine fluorescente verte, bêta-galactosidase et chloramphénicol acétyltransférase), un acide nucléique sélectionné parmi un ARN, un ADN, un ADNc, les oligonucléotides antisens et un ARNsi, un glucide ou un lipide, pour délivrer la cargaison de l'objet dans une cellule.

2. Utilisation *in vitro* d'un support de délivrance intracellulaire dans lequel une cargaison d'un objet à délivrer dans une cellule est conjuguée à l'extrémité d'un peptide NC d'un rétrovirus, dans laquelle la cargaison de l'objet à délivrer dans une cellule est une substance chimique sélectionnée parmi un médicament, un agent de contraste (par exemple, agent de contraste T1, agent de contraste T2 tel qu'une substance superparamagnétique, un radioisotope, etc.), un marqueur fluorescent, et un agent colorant, un polypeptide sélectionné parmi les protéines qui sont impliquées dans l'immortalisation cellulaire (par exemple, grand antigène T de SV40 et télomérase), les protéines anti-apoptotiques (par exemple, p53 mutante et BclxL), les anticorps, les gènes de cancer (par exemple, ras, myc, HPV E6/E7 et Ela d'adénovirus), les protéines de régulation du cycle cellulaire (par exemple, cycline et phosphorylase dépendante de la cycline) et les enzymes (par exemple, protéine fluorescente verte, bêta-galactosidase et chloramphénicol acétyltransférase), un acide nucléique sélectionné parmi un ARN, un ADN, un ADNc, les oligonucléotides antisens et un ARNsi, un glucide ou un lipide,
et dans laquelle le peptide NC est sélectionné dans le groupe constitué des polypeptides présentant la séquence d'acides aminés représentée par SEQ ID NO : 12 à SEQ ID NO : 15,
pour délivrer la cargaison de l'objet dans une cellule.

3. Utilisation selon la revendication 2, dans laquelle le rétrovirus est sélectionné dans le groupe constitué du virus de l'immunodéficience humaine (VIH), du virus de la leucémie murine (MLV), du virus de l'immunodéficience simienne (SIV) et du RSV (virus du sarcome de Rous).

4. Procédé *in vitro* de délivrance d'une cargaison d'un objet à délivrer dans une cellule, le procédé comprenant la mise en contact d'une cellule avec un support de délivrance intracellulaire dans lequel la cargaison de l'objet à délivrer dans la cellule est conjuguée à l'extrémité d'un peptide de pénétration cellulaire comprenant une séquence d'acides aminés représentée par l'une quelconque parmi SEQ ID NO : 17 à 23,
dans lequel la cargaison de l'objet à délivrer dans la cellule est une substance chimique sélectionnée parmi un médicament, un agent de contraste (par exemple, agent de contraste T1, agent de contraste T2 tel qu'une substance superparamagnétique, un radioisotope, etc.), un marqueur fluorescent, et un agent colorant, un polypeptide sélectionné parmi les protéines qui sont impliquées dans l'immortalisation cellulaire (par exemple, grand antigène T de SV40 et télomérase), les protéines anti-apoptotiques (par exemple, p53 mutante et BclxL), les anticorps, les gènes de cancer (par exemple, ras, myc, HPV E6/E7 et Ela d'adénovirus), les protéines de régulation du cycle cellulaire (par exemple, cycline et phosphorylase dépendante de la cycline) et les enzymes (par exemple, protéine fluorescente verte, bêta-galactosidase et chloramphénicol acétyltransférase), un acide nucléique sélectionné parmi un ARN, un ADN, un ADNc, les oligonucléotides antisens et un ARNsi, un glucide ou un lipide.

5. Procédé *in vitro* de délivrance d'une cargaison d'un objet à délivrer dans une cellule, le procédé comprenant la mise en contact d'un support de délivrance intracellulaire dans lequel une cargaison d'un objet à délivrer dans une cellule est conjuguée à l'extrémité d'un peptide NC d'un rétrovirus avec une cellule,
dans lequel la cargaison de l'objet à délivrer dans la cellule est une substance chimique sélectionnée parmi un médicament, un agent de contraste (par exemple, agent de contraste T1, agent de contraste T2 tel qu'une substance superparamagnétique, un radioisotope, etc.), un marqueur fluorescent, et un agent colorant, un polypeptide sélectionné parmi les protéines qui sont impliquées dans l'immortalisation cellulaire (par exemple, grand antigène T de SV40 et télomérase), les protéines anti-apoptotiques (par exemple, p53 mutante et BclxL), les anticorps, les gènes de cancer (par exemple, ras, myc, HPV E6/E7 et Ela d'adénovirus), les protéines de régulation du cycle cellulaire (par exemple, cycline et phosphorylase dépendante de la cycline) et les enzymes (par exemple, protéine fluorescente verte, bêta-galactosidase et chloramphénicol acétyltransférase), un acide nucléique sélectionné parmi un ARN, un ADN, un ADNc, les oligonucléotides antisens et un ARNsi, un glucide ou un lipide, et dans lequel le peptide NC est sélectionné dans le groupe constitué des polypeptides présentant la séquence d'acides aminés représentée par SEQ ID NO : 12 à SEQ ID NO : 15.

6. Procédé selon la revendication 5, dans lequel le rétrovirus est sélectionné dans le groupe constitué du virus de l'immunodéficience humaine (VIH), du virus de la leucémie murine (MLV), du virus de l'immunodéficience simienne (SIV) et du RSV (virus du sarcome de Rous).
